# EUROPÄISCHE PATENTSCHRIFT

(11) **EP 0 485 685 B1**
(45) Veröffentlichungstag und Bekanntmachung des Hinweises auf die Patenterteilung: **05.08.1998**
(21) Anmeldenummer: 91103198.7
(22) Anmeldetag: 04.03.1991
(51) Int. Cl.: C07D 223/22

(54) **Verfahren zur Herstellung von 5-Carbamoyl-5H-dibenz-[B,F]-azepin**
Process for the preparation of 5-carbamoyl-5H-dibenz-[B,F]-azepine
Procédé pour la préparation de la 5-carbamoyl-5H-dibenz-[B,F]-azépine

(30) Priorität: 05.07.1990 DD 342510
(43) Veröffentlichungstag der Anmeldung: 20.05.1992
(60) Teilanmeldung: 95117215.4
(73) Patentinhaber: Arzneimittelwerk Dresden GmbH, 01445 Radebeul (DE)
(72) Erfinder: Palitzsch, Peter, Dipl.-Chem., O-8023 Dresden (DE); Müller, Rainer, Dr., O-8019 Dresden (DE); Richter, Ehrhard, Dr., O-8122 Radebeul (DE)
(74) Vertreter: Grabherr, Claudia, Dipl.Ing.

(56) Entgegenhaltungen:
- CHEMICAL ABSTRACTS, Band 113, Nr. 1, 2. Juli 1990, Seite 600, Zusammenfassung Nr. 6185n, Columbus, Ohio, US; & PL-A-147 078 (POLFA) 29-04-1989
- CHEMICAL ABSTRACTS, Band 101, Nr. 22, 26. November 1984, Seite 365, Zusammenfassung Nr. 198028k, Columbus, Ohio, US; E. LAINE et al.: "Formation of dihydrate from carbamazepine anhydrate in aqueous conditions", & INT. J. PHARM. 1984, 20(3), 307-14

## Beschreibung

Die Erfindung betrifft ein technisches Verfahren zur Herstellung von 5-Carbamoyl-5H-dibenz/b,f/azepin (III), das als Arzneimittel, vorzugsweise als Antiepileptikum, verwendet wird.

Die Synthese des 5-Carbamoyl-5H-dibenz/b,f/azepins (III) aus Iminostilben (I) (=5H-Dibenz/b,f/azepin) wurde erstmalig von W. Schindler (DE-AS 1.136.707, CH-PS 54.023) beschrieben.

Nach diesem Verfahren wird das Iminostilben (I) in Toluen suspendiert, und in diese Suspension wird Phosgen eingeleitet, wobei sich die Reaktionsmischung bis auf 70°C erwärmt. Anschließend wird die Reaktionsmischung unter weiterem Einleiten von Phosgen zum Rückfluß angeheizt und so lange gekocht; bis das Iminostilben vollständig umgesetzt und die Chlorwasserstoffentwicklung beendet ist.

Sobald die Reaktionslösung frei von Iminostilben ist, wird die Phosgeneinleitung abgestellt. Das überschüssige Phosgen wird aus der Reaktionsmischung mit trockenem Stickstoff oder trockener Luft entfernt. (s. hierzu DE-AS 1.001.271, wonach das überschüssige Phosgen nach der beendeten Phosgenierung des 5H-10,11-Dihydro-dibenz/b,f/azepins = Iminodibenzyl gleichfalls mit trockener Luft ausgeblasen wird). Die derartig entgiftete Reaktionslösung wird wie üblich aufgearbeitet, und das durch Kristallisation isolierte 5-Chlorcarbonyl-5H-dibenz/b,f/azepin (II) wird in bekannter Weise zum 5-Carbamoyl-5H-dibenz/b,f/azepin (III) amidiert.

Die bis zum heutigen Zeitpunkt beschriebenen Verfahren arbeiten alle in inerten wasserfreien Lösungsmitteln bei Temperaturen über 100 °C ( beispielsweise in Toluen, in Chlorbenzen oder in o-Dichlorbenzen; siehe hierzu in Betracht gezogene Patentschriften und Übersichtsartikel von B.RENFROE, C.HARRINGTON und G.R. PROCTOR in "Heterocyclic compounds", Vol. 43, "Azepines", Teil I, John Wiley & Sons, New York 1984, Seite 524, Tabelle 118).

Die Dissoziation des bei der Phosgenierung stets entstehenden Iminostilben-Hydrochlorids in Chlorwasserstoffgas und freies Iminostilben wird bei allen technischen Verfahren thermisch vorgenommen; indem man das Reaktionsgemisch bis zum Siedepunkt des indifferenten Lösungsmittels anheizt und unter Rückfluß Phosgen einleitet.

Die nach dem Stand der Technik durchgeführten Heißtemperaturphosgenierungen arbeiten alle bei 100°C und höher; um eine vollständige Phosgenierung des Iminostilbens oder Iminodibenzyls zu erreichen.

Die bekannten Verfahren zur Herstellung von Carbamidsäurechloriden aus sekundären Aminen sind in Houben-Weyl ( Band E 4; Seite 46 - 50, 1983) in einer Tabelle zusammengestellt. Als Lösungsmittel werden in der Regel vor allem aromatische Kohlenwasserstoffe wie Benzen, Toluen oder Chlorbenzen eingesetzt.

Arbeitet man bei niederen Temperaturen, so wird bei der Einleitung von Phosgen in eine Lösung des sekundären Amins in einem inerten Lösungsmittel nur die Hälfte des Amins in das gewünschte Carbamidsäurechlorid überführt, weil der bei der Reaktion freiwerdende Chlorwasserstoff die andere Hälfte des Amins in das Hydrochlorid überführt. Das Amin-Hydrochlorid scheidet sich in kristalliner Form ab, d.h. die Ausbeute an Carbamidsäurechlorid kann im günstigsten Fall nur 50% betragen.

Seit der Arbeit von H.ERDMANN und P.HUTH [ J.Prakt.Chem.(2) 56, 7(1897)] ist bekannt, daß man den Umsatz vervollständigen kann, wenn man eine inerte wasserfreie Base wie Pyridin in mindestens äquimolekularer Menge zusetzt. Nach HOUBEN-WEYL (s.o.) sind als inerte Basen außer Pyridin noch Triethylamin und naturgemäß das umzusetzende Amin selbst geeignet. Da man stets mindestens äquimolekulare Mengen an inerter Base benötigt, verteuert sich die Kaltphosgenierung und der Verfahrensablauf wird technologisch aufwendig, denn das Amin-Hydrochlorid muß auf jeden Fall zwecks Rückgewinnung der inerten Base abgetrennt werden.

Aus diesem Grund besitzt die Kaltphosgenierung in Gegenwart von inerten Hilfsbasen nur Bedeutung für die Umsetzung von temperaturempfindlichen sekundären Aminen, die bei den hohen Temperaturen der Heißphosgenierung verstärkt zu unerwünschten Nebenreaktionen neigen.

In der Technik arbeitet man bevorzugt bei Temperaturen oberhalb von 100°C. Im Houben-Weyl(s.o.) wird hierzu ausgeführt:
"Vorteilhafterweise erhitzt man das Reaktionsgemisch unter weiterem Einleiten von Phosgen auf über 100°C, wobei das Amin-Hydrochlorid in das Carbamidsäurechlorid übergeht."

Das bei dieser thermischen Dissoziation freiwerdende HCl- Gas reißt beträchtliche Mengen Phosgen mit. Aus diesem Grund muß das Abgas in speziellen Abgasanlagen entgiftet und vernichtet werden. Im Haveriefall kann eine derartige Verfahrensweise zu einer ernsthaften Gefährdung der Umwelt führen. (Aufgrund der extremen Giftigkeit des Phosgens besteht vor allem eine Gefahr für angrenzende Wohngebiete.)

Die Arbeitsweise der Heißphosgenierung hat vor allem die folgenden schwerwiegenden Nachteile:
- hohe Abgasbelastungen durch freigesetztes HCl-Gas, durch mitgerissenes Phosgen und mitgeführte Lösungsmitteldämpfe und die daraus resultierenden Umweltschutzprobleme
- lange Reaktionszeiten über 18-24 Stunden mit starken korrosiven Belastungen der Anlagen
- hohe energetische Aufwendungen und
- eine Vielzahl von Nebenreaktionen sowie Dunkelfärbung des Reaktionsproduktes, die letzten Endes zu entscheidenden Qualitätsminderungen des 5-Carbamoyl-5H-dibenz/b,f/azepins führen sowie die
- oberhalb von 90°C verstärkt stattfindende Verengung des Siebenringes des Iminostilbens unter Bildung von unerwünschtem 9-Methylacridin.

Das Iminostilben ist in die Gruppe der temperaturempfindlichen Amine einzuordnen. Aus diesen Gründen wird Iminostilben vorteilhafterweise nach dem Verfahren von SCHINDLER (DE-AS 1.136.707) phosgeniert.

Die thermische Dissoziation des Iminostilben-Hydrochlorids in freies Iminostilben und HCl-Gas erfolgt erst bei ca. 90°C hinreichend schnell, um für industrielle Zwecke akzeptable Reaktionszeiten zu erreichen.

Die von SCHINDLER bevorzugte Variante der Zweiteilung der Phosgenierung in eine Phase der Kaltphosgenierung (Umsatz der ersten Hälfte, 50%) und eine Phase der Heißphosgenierung ( Umsatz der zweiten Hälfte) besitzt gegenüber der direkten Heißphosgenierung eindeutige Vorteile , denn erstens wurde auf diese Weise die Ausbeute beträchtlich erhöht , zweitens wurden die oberhalb von 90°C ablaufenden Nebenreaktionen zurückgedrängt und drittens wurden die Qualität und die Farbe verbessert. Trotzdem bleiben aber die beschriebenen Nachteile in der zweiten Reaktionsstufe - von Beginn des Anheizens auf 90°C und während der thermischen Dissoziation des Iminostilben-Hydrochlorids bis zum Endpunkt der Reaktion - erhalten.

Um die schonenden Reaktionsbedingungen der Kaltphosgenierungsphase möglichst vollständig auszunutzen, wird ein Phosgenüberschuß in die Reaktionsmischung eingeleitet. Heizt man anschließend die Reaktionsmischung zur thermischen Dissoziation des Iminostilben-Hydrochlorids an, so kann es zum Druckstoß kommen.

Auf diese Gefahr wird im HOUBEN-WEYL (Band E4 ,Seite 744, 1983) ebenfalls aufmerksam gemacht.

Bei einem Druckstoß reißen die spontan freigesetzten HCl-Gasmengen beträchtliche Mengen Phosgen mit. Die Abgas- Vernichtungsanlage bzw. -Entgiftungsanlage muß folglich groß genug dimensioniert sein, um einen derartigen Druckstoß des Phosgens in die Atmosphäre zu verhindern.

Um die unerwünschten Nebenreaktionen und die Bildung von 9-Methylacridin zurückzudrängen, arbeitet man zweckmäßig bei Temperaturen von 90-100°C. Bei dieser Reaktionsführung verläuft zwar die Phosgenierung hinreichend schnell, aber der Dampfdruck des Phosgens ist gegenüber der Phase der Kaltphosgenierung naturgemäß beträchtlich erhöht, so daß es nicht zu verhindern ist, daß der freigesetzte Chlorwasserstoff ständig große Mengen Phosgen mitführt. Dies äußert sich schon in der Tatsache , daß für den Umsatz der ersten Hälfte Iminostilben in der Phase der Kaltphosgenierung wesentlich weniger Zeit benötigt wird als für den Umsatz der zweiten Hälfte in der Phase der Heißphosgenierung.

Nach dem vollständigen Umsatz des Iminostilbens muß die Reaktionslösung entgiftet werden. In der Regel wird das überschüssige Phosgen aus der heißem Reaktionslösung mit trockenem Stickstoff ausgeblasen, oder es wird ein Teil des Lösungsmittels abdestilliert, bis die Reaktionsmischung phosgenfrei ist. Diese Verfahrensweisen der Entgiftung besitzen den Nachteil, daß bei Undichtigkeiten aufgrund des in der Anlage herrschenden Gasdruckes Phosgen austreten kann. Über längere Zeiträume besteht folglich die Gefahr, daß die Umwelt durch Phosgen belastet werden kann.

In der Literatur sind außer dem beschriebenen Verfahren zur direkten Phosgenierung des Iminostilbens ( I) noch einige Verfahren zur Synthese des 5-Carbamoyl-5H-dibenz/b,f/ azepins (III) beschrieben worden, die vom Iminodibenzyl(IV) (=10,11-Dihydro-5H-dibenz/b,f/azepin) ausgehen (siehe hierzu Patentschriften GB 1.246.606, DD 82.719, DD 100.948, DD 101.671, DD 102.149, DD 102.150, DD 102.151, DD 108.535, DD 133.052, DD 234.862 A1, DD 234.863 A1 ) Nach diesem Verfahren wird Iminodibenzyl ( IV) in einem siedenden aromatischen Lösungsmittel -vorzugsweise Toluen oder Chlorbenzen- mit Phosgen umgesetzt, wobei das Phosgen in den Rücklauf eingeleitet wird. Es handelt sich folglich wieder um eine Heißphosgenierung mit allen ihren Nachteilen.

Das erhaltene 5-Chlorcarbonyl-5H-10,11-dihydro-dibenz/b,f/azepin (V) wird anschließend in einem inerten organischen Lösungsmittel mit elementarem Brom oder einem selektiven Bromierungsreagenz umgesetzt, wobei die entsprechenden 10-Monobrom ( VI, R=H) und/oder 10,11-Dibrom-Derivate ( VII, R= Br) gebildet werden . Anschließend werden die Brom-Verbindungen (VI und/oder VII) thermisch dehydrobromiert und /oder debromiert . Bei diesem thermischen Prozeß erfolgt gleichzeitig ein teilweiser Austausch ( 30- 40% ) des Chloratoms der 5-Chlorcarbonyl-Gruppe gegen ein Brom-Atom.

Diese drastischen Reaktionsbedingungen führen zwangsläufig aufgrund der erforderlichen hohen Reaktionstemperaturen ( 150 bis 170°C ) und des freiwerdenden Broms zu nicht kontrollierbaren Nebenreaktionen (Kernbromierungen, Verharzungen, Verfärbung, Crackung).

Daraus folgt, daß das über derartige Prozesse synthetisierte 5-Chlorcarbonyl-5H-dibenz/b,f/azepin (II) außer zahlreichen -vor allem bromhaltigen - Nebenprodukten noch schmierige teerige Anteile und Farbstoffe enthält, deren Entfernung einen enormen Aufwand erfordert bzw. bei durchgängigem Arbeiten auf 5-Carbamoyl-5H-dibenz(b,f/azepin (III) zwangläufig zu Qualitätseinbußen beim Finalprodukt führt.

Über die Natur und Struktur der erwähnten Nebenprodukte liegen keine Kenntnisse vor. Die üblichen Reinigungsverfahren führen zu beträchtlichen Verlusten.

Bisher existiert kein Reinigungsverfahren, daß in ökonomisch vertretbarer Weise das Problem des Restbromgehaltes löst. Das alles ist erklärbar, denn der laut HPLC ermittelte Gehalt des auf diese Weise synthetisierten 5-Chlorcarbonyl-5H-dibenz/b,f/-azepins (II) liegt durchschnittlich bei 90%, d.h. das Vorprodukt-für das Finalprodukt Carbamazepin- enthält nach dem beschriebenen Verfahren 10% Verunreinigungen.

Das nach den oben beschriebenen Verfahren hergestellte 5-Chlorcarbonyl-5H-dibenz/b,f/azepin wird als Substanz isoliert, indem man entweder die heiße Lösung mit Aktivkohle behandelt, filtriert und das Produkt nach der Kristallisation isoliert oder indem man nach der Filtration das Lösungsmittel abdestilliert, die Schmelze des Produktes in ein Fällbad ( in ein anderes geeignetes Lösungsmittel) einlaufen läßt und anschließend das kristallisierte Produkt abtrennt.

Das substantiell isolierte 5-Chlorcarbonyl-5H-dibenz/b,f/azepin wird in einem geeigneten Lösungsmittel amidiert, wobei sowohl NH₃ Gas bzw. Flüssig-NH₃ als auch konzentriertes Ammoniakwasser oder Ammoniumsalze in wäßriger Lösung zum Einsatz kommen.

In der Patentliteratur werden folgende Amidierungsverfahren beschrieben.

Nach den Verfahren DD-PS 82.719, DD-PS 102.150, CH-PS 366.541 und DE-AS 1.001.271 wird das 5-Chlorcarbonyl-5H-dibenz/b,f/-azepin in Ethanol oder Methanol teilweise oder vollständig gelöst. Die Suspension bzw. Lösung wird mit gasförmigem Ammoniak bei Temperaturen behandelt, die über dem Siedepunkt des betreffenden Alkohols liegen, d.h. die Amidierung erfolgt als Druckreaktion in einem Autoklaven. Diese Problematik ist technisch zwar beherrschbar, aber schwerer wiegt der Umstand, daß die eingesetzten Alkohole sich nicht inert verhalten, sondern in unerwünschtem Maß mit dem 5-Chlorcarbonyl-5H-dibenz/b,f/azepin in einer nicht zu unterdrückenden Nebenreaktion reagieren. Als Nebenprodukte entstehen die betreffenden Ester.

Bei der Amidierung in Methanol ( wobei es gleichgültig ist, ob man wasserfrei mit NH₃-Gas oder mit konzentriertem Ammoniakwasser arbeitet ) bilden sich etwa 1-2% 5-Carbomethoxy-5H-dibenz/b,f/azepin. Dieser Ester ist bei der nachfolgenden Umkristallisation des rohen 5-Carbamoyl-5H-dibenz/b,f/ azepins nicht ohne weiteres entfernbar. Selbst nach zweimaliger Umkristallisation enthält das Endprodukt noch 0,1 % des 5-Carbomethoxy-5H-dibenz/b,f/-azepins. Da nach der europäischen Pharmakopoe die maximale Einzelverunreinigung 0,01% nicht überschreiten darf, führen alle Amidierungsverfahren in alkoholischen Lösungsmitteln (vor allem mit primären niederen Alkoholen ) zu einem nicht qualitätsgerechtem Carbamazepin. Diese Amidierungsverfahren scheiden - nach den heutigen Qualitätsanforderungen - aus der Betrachtung aus.

Nach dem Verfahren DD-PS 82.719 kann die Amidierung auch in einem inerten aromatischen Lösungsmittel ( Beispiel 1, Amidierung von 33,7g 5-Chlorcarbonyl-10-brom-iminodibenzyl in 500 ml Benzen im Autoklav) vorgenommen werden. Diese Variante hat den Vorteil, daß keine Esterbildung stattfinden kann. Da das gebildete Carbamazepin aber aufgrund seiner Polarität in Benzen oder Toluen selbst in der Hitze schlecht löslich ist, benötigt man aus Gründen der Rührfähigkeit und Durchmischung und der damit verbundenen - unbedingt erforderlichen - quantitativen Reaktion des Carbamidsäurechlorides wesentlich mehr Lösungsmittel .

Dadurch sinkt die Raum-Zeit-Ausbeute drastisch ab.

Nach dem Verfahren der DD-PS 264.115 A3 wird die Amidierung ebenfalls in einem aromatischen Lösungsmittel vorgenommen, wobei man die Lösung des 5-Halogencarbonyl-5H-dibenz/b,f/azepins in das Amidierungsmittel einlaufen läßt.

Als Amidierungsmittel dienen 15 - 25% iges Ammoniakwasser oder wäßrige Ammoniumsalzlösungen. Um in konzentrierter Form arbeiten zu können, werden nichtionogene Tenside oder quartäre Aryl-alkylammoniumsalze als grenzfächenaktive Stoffe zugesetzt. Diese Stoffe sollen eine bessere Durchmischung und Rührbarkeit zwecks Erzielung des quantitativen Umsatzes gewährleisten. Vorzugsweise werden Halogenaromaten wie beispielsweise Chlorbenzen oder Brombenzen eingesetzt. An dieser Stelle ist einzuwenden, daß aus Umweltschutzgründen (Abwasserbelastung! ) der Trend eindeutig zu Verfahrensentwicklungen geht, die ohne Chlorkohlenwasserstoffe auskommen.

Es scheiterten alle Versuche, das beschriebene Verfahren DD-PS 264.115 A3 mit Toluen als Lösungsmittel ( Benzen scheidet aus , weil es Blutkrebs erzeugt) in die industrielle Großproduktion zu überführen. Das Reaktionsgemisch ist nach spätestens einer Stunde in herkömmlichen Rührmaschinen ( 3200 l Rührmaschine, Impellerrührer mit 103 U/min, oder mit Ankerrührer ) nicht mehr rührfähig und nicht durchmischbar. Die Reaktion kommt bereits weit vor dem erforderlichen vollständigen Umsatz zum Stillstand, weil das ausgeschiedene gebildete 5-Carbamoyl-5H-dibenz/b,f/azepin nach oben treibt und einen ca. 1m starken zusammenhängenden " Kristallkuchen" bildet, der sich nicht mehr verrühren läßt. Versucht man die Masse durch Temperaturerhöhung ( Anheizen auf ca 105-110°C bei steigendem Druck, System geschlossen) rührfähig zu bekommen , so gelingt dies zwar im Verlauf von 1-2 Stunden , aber die Temperaturerhöhung hat zur Folge, daß das noch vorhandene unumgesetzte 5-Chlorcarbonyl-5H-dibenz/b,f/azepin hydrolysiert - also zersetzt - wird. Auf diese Weise werden bis zu 8% der eingesetzten 5-Chlorcarbonyl-iminostilben-Menge in das goldgelbe Ausgangsprodukt Iminostilben überführt. Dadurch wird das gebildetet Carbamazepin verfärbt. Die Reinigung gestaltet sich aufwendig, und die Kosten steigen beträchtlich an.

Aus den gleichen Gründen konnte auch das vorgeschlagene Verfahren WP C07D/321.613 5 (das auf dem Verfahren WP C07D 320.612 aufbaut) nicht erfolgreich in die Großproduktion überführt werden.

Somit steht nach wie vor kein durchgängiges, kostengünstiges Verfahren zur Herstellung von 5-Carbamoyl-5H-dibenz/b,f/azepin zur Verfügung, wonach ein Produkt gemäß den Anforderungen des "Europäischen Arzneibuches" erhalten werden kann.

Als zur Zeit gültiges und betriebenens Verfahren verbleibt die Amidierung nach DD-PS 126.329. Nach diesem Verfahren wird das isolierte 5-Chlorcarbonyl-5H-dibenz/b,f/azepin in Butylacetat eingetragen und mit konzentriertem Ammoniakwasser amidiert. Obwohl diese Reaktion in einem Zweiphasensystem abläuft, kann auch hier die Hydrolyse des 5-Chlorcarbonyl-5H-dibenz/b,f/azepins nicht vollständig unterdrückt werden. Eine gewisse "Rückreaktion" zum Iminostilben ist - je nach den gewählten Reaktionsparametern - immer mehr oder weniger zu beobachten.

Die Blockierung der Rührmaschine in der oben genannten Weise findet ebenso bei dem Verfahren gemäß PL-A-147 078 statt, bei dem die Amidierung des 5-Chlorcarbonyl-5H-dibenzo /b,f/ azepins in wässriger Ammoniaklösung durchgeführt wird.

Aus Chemical Abstracts, Bd. 101, Nr. 22, 1984, Art.Nr. 198028k (Laine et al) ist es bekannt, daß Carbamazepin unter wässrigen Bedingungen Carbamazepin-dihydrat als Haarkristalle bildet. Das Vorliegen dieser Haarkristalle insbesondere in einem Gemisch mit anderen Stoffen u.a. Ammoniumchlorid beurteilt der Fachmann als Problem bei den folgenden Trennoperationen.

Die Erfindung verfolgt das Ziel, 5-Carbamoyl-5H-dibenz/b,f/azepin der Formel (III) unter äußerst schonenden und milden Reaktionsbedingungen in nahezu quantitativer Ausbeute bei wesentlich verbesserter Qualität in einem durchgehenden Verfahren ohne Isolierung der Zwischenstufe 5-Chlorcarbonyl-5H-dibenz/b,f/azepin (II) direkt aus Iminostilben (I) herzustellen, wobei das neuartige Verfahren mit wesentlich verkürzter Reaktionszeit und mit erheblich geringerem Energiebedarf auskommt. Mit der Erfindung sollen ferner eine deutliche Verbesserung des Umweltschutzes und merklich verringerte Gefährdungsmomente erzielt werden.

Der vorliegenden Erfindung liegt die Aufgabe zugrunde, 5-Carbamoyl -5H-dibenz/b,f/azepin der Formel (III) bei verkürzter Reaktionszeit unter Vermeidung der Heißphosgenierungsphase bzw. unter Ausschaltung der bisher üblichen thermischen Dissoziation des Iminostilben-Hydrochlorids in quantitativer Ausbeute und in wesentlich verbesserter Qualität in einem durchgehenden Verfahren direkt aus Iminostilben ohne Isolierung der Zwischenstufe 5-Chlorcarbonyl-5H-dibenz/b,f/azepin und ohne Wechsel des Lösungsmittels herzustellen.

Die direkte Herstellung des Finalproduktes der Formel (III) ohne substantielle Isolierung und Vorreinigung des sehr hauttoxischen 5-Chlorcarbonyl-5H-dibenz/b,f/azepins der Formel (II) in einem durchgehenden Verfahren soll ermöglicht werden durch einen quantitativen Umsatz des Iminostilbens (I) zu 5-Chlorcarbonyl-5H-dibenz/b,f/azepin (II), wobei das Zwischenprodukt der Formel (II) in so hervorragender Qualität anfällt, daß eine Isolierung oder eine Reinigungsoperation über ein Fällbad oder eine Kristallisation in Wegfall kommen kann.

Gemäß dem bereits in der Anmeldung WP C07D/320.612 vorgeschlagenen Verfahren wird in eine Suspension von Iminostilben (I) in einem inerten aromatischen Lösungsmittel bei 20 - 60 °C Phosgen eingeleitet, oder man läßt eine Lösung von Phosgen im gleichen Lösungsmittel zulaufen , bis das Iminostilben zu einem nahezu äquimolekularen Gemisch aus 5-Chlorcarbonyl-5H-dibenz/b,f/azepin (II) und Iminostilben-Hydrochlorid umgesetzt ist, worauf man durch Zugabe einer wäßrigen Base aus dem gebildeten Iminostilben-Hydrochlorid Iminostilben freisetzt und so bei Aufrechterhaltung eines sauren Reaktionsmilieus der vollständigen Phosgenierung zugänglich macht.

Vorzugsweise wird bei der Phosgenierung im Temperaturbereich von 35-60°C gearbeitet. Nach dem etwa 50%igen Umsatz des Iminostilbens zu dem gewünschten Carbamidsäurechlorid (II) beginnt man unter ständigem weiteren Einleiten von Phosgen entweder mit dem Zulauf einer verdünnten Lösung eines Alkalihydroxides oder von verdünntem Ammoniakwasser oder einer wäßrigen Lösung eines Alkalicarbonates bzw. -hydrogencarbonates oder einer wäßrigen Lösung eines infolge Hydrolyse alkalisch reagierenden Salzes (beispielsweise Natriumacetat).

Während des Zulaufes der wäßrigen Base wird die Temperatur bei 35 - 60°C gehalten.

Nach dem Zulauf der wäßrigen Base wird die Einleitung des Phosgens bis zum 100%igen Umsatz des Iminostilbens fortgesetzt, wobei die angegebenen Temperaturgrenzen eingehalten werden . Der vollständige Umsatz des Iminostilbens wird dünnschichtchromatographisch ermittelt.

In der Endphase der Phosgenierung hält man die Temperatur bei etwa 40-50°C (in Chlorbenzen) bzw. bei 50-60°C (in Toluen, wobei leicht höhere Temperaturen mitunter angewandt werden müssen, um das gebildete 5-Chlorcarbonyl-5H-dibenz/b,f/azepin in Lösung zu halten) , damit das gebildete Carbamidsäurechlorid (II) nicht auskristallisiert.

Temperaturabweichungen um einige Grad nach oben oder unten besitzen keine Bedeutung.

Sobald in der Reaktionslösung kein Iminostilben mehr nachweisbar ist, wird die Phosgeneinleitung beendet. Danach wird die stark saure Reaktionsmischung (die den pH-Wert 1 besitzt) langsam auf 80 -90°C erwärmt, so daß das überschüssige Phosgen durch die in der wäßrigen Phase vorhandene Salzsäure hydrolysiert wird. Auf diese Weise wird die Reaktionsmischung in wesentlich kürzerer Zeit und ungefährlicher entgiftet als nach dem Ausblasverfahren der unter Ausschluß von Wasser durchgeführten Heißphosgenierung.

Weder das bereits gebildete 5-Chlorcarbonyl-5H-dibenz/b,f/azepin(II) noch das eingeleitete Phosgen werden durch die zugesetzte wäßrige Base oder die gegen Ende der Phosgenierung vorliegende stark salzsaure wäßrige Phase in irgendeiner Form merklich zersetzt. Phosgen wird beispielsweise mit 15-20%iger Natronlauge entgiftet
( s."Organikum", 6.Auflage, S. 630, 1967). Das 5-Chlorcarbonyl- 5H-dibenz/b.f/azepin (II) wird als alkaliempfindliches Carbamidsäurechlorid ebenfalls in Gegenwart von verdünnten wäßrigen Alkalilaugen sofort spontan über das Natriumsalz der betreffenden Carbamidsäure unter Decarboxylierung wieder in Iminostilben überführt.

Das sind die Gründe , warum man Amine normalerweise nicht in Gegenwart wäßrig-alkalischer Lösungen mit Phosgen umsetzen kann.

In völlig unerwarteter Weise verläuft die Phosgenierung des Iminostilbens unter den beschriebenen Bedingungen außerdem wesentlich schneller und unter deutlich milderen, schonenderen Bedingungen. Die Reaktionszeit wird nach dem beschriebenen Weg auf die Hälfte verkürzt. Die Bildung der Nebenprodukte wird ebenfalls stark zurückgedrängt. Unter den beschriebenen Reaktionsbedingungen wird erstaunlicherweise nur sehr wenig 9-Methylacridin gebildet. Dieses unerwünschte Nebenprodukt wird nach dem beschriebenen Verfahren auf sehr elegante Art vorteilhafterweise nach der "Selbstentgiftung" bei der Phasentrennung entfernt. Die entgiftete heiße Reaktionslösung bleibt zur Phasentrennung bei 80-90 °C eine gewisse Zeit stehen. Die untere wäßrige stark saure Phase wird abgetrennt und verworfen. In dieser wäßrigen Phase sind -außer dem jeweiligen Salz- ungefähr 0,2% der ursprünglich eingesetzten Iminostilbenmenge in Form der verschiedensten Nebenprodukte enthalten. Davon entfallen schätzungsweise 50% auf 9-Methylacridin, das als Hydrochlorid vorliegt.

Die Entgiftung der Reaktionslösung durch die gebildete 7-10%ige Salzsäure (die jedoch bei übermäßiger Einleitung von Phosgen sogar maximal konzentriert sein kann) besitzt folglich noch einen positiven Effekt, indem basische aminartige Nebenprodukte in Form ihrer Hydrochloride aus der organischen Phase extrahiert werden, d.h. die wäßrig -saure Phase wirkt in bezug auf das Endprodukt 5-Carbamoyl-5H-dibenz/b,f/azepin (III) im Sinne einer Vorreinigung.

Erwünschtenfalls kann die Reaktionsmischung zur weiteren Reinigung noch einmal mit halbkonzentrierter Salzsäure extrahiert und nach der Phasentrennung auf den pH-Wert 5-6 abgepuffert und danach mit Adsorptionsmitteln wie z.B. Aktivkohle und/oder Aluminiumoxid behandelt werden.

Die beispielsweise kohlefrei filtrierte -vorzugsweise toluenische Lösung des 5-Chlorcarbonyl-5H-dibenz/b,f/azepins (II) wird durch aceotrope Destillation entwässert (Wasserabscheider).

Die trockene Lösung des Carbamidsäurechlorides (II) wird mit Lösungsmittel ergänzt bzw. verdünnt, so daß beispielsweise im Fall Toluen ein Verhältnis 5-Chlorcarbonyl-5H-dibenz/b,f/azepin (II) : Lösungsmittel von 1:6 (kg:l) eingehalten wird- als Vorzugsvariante.

In die filtrierte toluenische Lösung des 5-Chlorcarbonyl-5H-dibenz/b,f/azepins (II) wird bei 70-105°C Ammoniakgas eingeleitet, wobei der Innendruck bei 0,01-0,50 MPa , vorzugsweise bei 0,01 - 0,25 MPa, gehalten wird. In der Anfangsphase wird mit der Gaseinleitung bei ca. 70-80°C begonnen. Da die Reaktion exotherm ist, steigen die Temperatur und der Innendruck mit der Zeit an. Durch kurzzeitige Kühlwassergaben kann die Reaktion (Temperatur und Druck) ausgezeichnet gesteuert werden. Gegen Ende der Reaktion wird der Innendruck auf ca. 0,1- 0,25 MPa erhöht.

Nach dem mit der Anmeldung WP C07D 320 613 5 vorgeschlagenen Verfahren wird die Reaktionsmischung mit fortschreitender Amidierung immer sämiger, immer dicker, weil das gebildete Carbamazepin -Ammoniumchlorid-Gemisch auch bei höheren Temperaturen (bis 105 °C) sehr schlecht in Toluen löslich ist. Stellt man den Rührer ab, so erfüllt der dick-sämige Brei den gesamten Reaktionsraum . Die Kristalle setzen sich auch nach mehreren Stunden nicht ab. Aufgrund dieser Konsistenz verlangsamt sich gegen Ende der Amidierungsreaktion der Umsatz der restlichen 5% 5-Chlorcarbonyl-5H-dibenz/b,f/azepin (II) erheblich. Die Amidierungsreaktion läßt sich nicht so vervollständigen, daß das isolierte 5-Carbamoyl-5H-dibenz/b,f/azepin (III) ( =Carbamazepin) frei von 5-Chlorcarbonyl-5H-dibenz/b,f/azepin(II) ist. Das isolierte rohe Carbamazepin (III) muß jedoch frei von Carbamidsäurechlorid (II) sein , weil sich die letztere Verbindung bei der nachfolgenden Endreinigung des Carbamazepins (Umkristallisation) nicht mehr entfernen läßt. Die verschärften Qualitätsbestimmungen des "Europäischen Arzneibuches" lassen als maximale Einzelverunreinigung nur 0,01% zu. Nach dem erfindungsgemäßen Verfahren wirkt sich jedoch der Zusatz einer ganz begrenzten Menge Wasser an dieser Stelle der Reaktion überraschenderweise außerordentlich günstig auf das gesamte Rührverhalten, auf die Konsistenz der Reaktionsmischung durch einsetzende Kristallumwandlung und damit auf den vollständigen Umsatz sowie auf die Beschleunigung des Endumsatzes aus.

Hat man 100-110% der theoretischen Menge NH₃-Gas eingeleitet, so ändern sich die Temperatur und der Innendruck bei abgestellter NH₃-Zufuhr nicht mehr wesentlich. Die letzten 1-5% 5-Chlorcarbonyl-5H-dibenz/b,f/azepin (II) bringen keine große Wärmetönung mehr. Das aufgedrückte NH₃-Gas reagiert aufgrund des dick-sämigen Zustandes der Reaktionsmasse nur noch extrem langsam weg.

Setzt man jedoch beispielsweise 25 Gewichtsprozent Wasser [bezogen auf das eingesetzte 5-Chlorcarbonyl-5H-dibenz/b,f/azepin(II)] bei 85-95°C hinzu, so wird die Reaktionsmischung in kurzer Zeit zusehends dünnflüssiger und hervorragend rührfähig. Stellt man den Rührer ab , so sinken die Kristalle unter, und es trennt sich innerhalb kurzer Zeit die toluenische Lösung als " klare Oberphase" ab. Überraschenderweise fällt auch die Temperatur der Reaktionsmischung erheblich ab ( weit über das berechnete Maß), wenn die begrenzte Menge Wasser zugesetzt wird. Es wird angenommen, daß eine Kristallstrukturumwandlung stattfindet, die Energie verbraucht.

Aus dem dick-sämigen Brei bildet sich ein sandartiges Kristallisat, das so schwer ist, daß es sich bei abgestelltem Rührer absetzt. Durch die auf diese Weise wesentlich verbesserten Rührbedingungen und die dünnflüssige Konsistenz der Reaktionsmischung gelingt es , den Umsatz zu vervollständigen.

Man drückt noch 10-20 % Überschuß in Bezug auf die theoretisch benötigte NH₃-Menge nach. Anschließend rührt man die Reaktionsmischung weitere 3-5 Stunden bei 80-95°C, wobei man gegen Ende der Nachreaktionszeit den Reaktor ( über eine Absorptionsanlage) entspannt, unter Rückfluß (aceotroper Siedepunkt liegt anfangs bei ca. 82°C, etwas niedriger als üblich durch entweichendes NH₃-Gas) entgast und ausreagieren läßt. Die Endpunktbestimmung erfolgt dünnschichtchromatographisch, wobei die abgekühlte klare Toluen-Mutterlauge gegen eine Vergleichslösung von 5-Chlorcarbonyl-5H-dibenz/b,f/azepin (II) in Toluen aufgetragen wird. In der Toluen-Mutterlauge darf höchstens 1% 5-Chlorcarbonyl-5H-dibenz/b,f/azepin(II) enthalten sein. D.h. der Umsatz muß auf mindestens 99% der Theorie gebracht werden. Das abgesaugte 5-Carbamoyl-5H-dibenz/b,f/azepin(III) enthält kein 5-Chlorcarbonyl-5H-dibenz/b,f/azepin(II).

Nach dem Ende der Amidierungsreaktion wird die Reaktionsmischung abgekühlt. Saugt man das rohe 5-Carbamoyl-5H-dibenz/b,f/-azepin (III) ab ,so stellt man überaschenderweise fest, daß die Toluen-Mutterlauge trotz des Wasserzusatzes trocken ist, d.h. unter dem Toluen sitzt keine wäßrige Phase.

Daraus folgt, daß das abgesaugte Kristallisat ( das sich ausgezeichnet absaugen läßt) ein Mischkristallisat aus 5-Carbamoyl-5H-dibenz/b,f/-azepin(III) und Ammoniumchlorld darstellt, das das zugesetzte Wasser vollständig bindet.

Von Vorteil ist, daß das Mischkristallisat toluenabweisend ist. Verrührt man das Mischkristallisat mit der 5-8fachen Menge Wasser bei 50-60°C mehrere Stunden, um das Ammoniumchlorid herauszulösen; so quillt das rohe 5-Carbamoyl-5H-dibenz/b,f/-azepin (III) zwar auf, aber es läßt sich gut verrühren und nach dem Abkühlen sehr gut absaugen oder zentrifugieren. Saugt man das Rohprodukt nach dem Abkühlen ab, so ist auf der wäßrigen Ammoniumchloridlösung keine Toluenphase zu beobachten.

Diese im Prinzip nahezu quantitative Trennung der beiden Lösungsmittel Toluen und Wasser ist von Vorteil für den technologischen Ablauf der industriellen Großsynthese.

Nach dem erfindungsgemäßen Verfahren können 3200 l Rührmaschinen ( oder größer) mit Impellerrührer (103 Umdrehungen /Minute;Abmessungen der 3200 l Rührmaschine : 1600 mm Innendurchmesser und 2150 mm Höhe) genutzt werden.

Im Gegensatz zu diesem Verfahren schlugen alle Versuche zur vollständigen Amidierung von 5-Chlorcarbonyl-5H-dibenz/b,f/azepin (II) nach den Verfahrensbeschreibungen der Patentschriften DD 264 115 A3 und WP C07D 320 613 5 in der gleichen Rührmaschine mit Impellerrührer fehl.

Sowohl mit konzentriertem Ammoniakwasser ( 110 l auf 100kg 5-Chlorcarbonyl-5H-dibenz/b,f/azepin) als auch allein mit NH₃-Gas unter Ausschluß von Wasser erhält man eine dicke entweder überhaupt nicht oder nicht sonderlich gut rührfähige Reaktionsmasse ( die weder mit einem Impellerrührer noch mit einem Ankerrührer in Rührmaschinen obiger Abmessungen bewegbar bzw. gut durchmischbar ist ), wodurch die unbedingt notwendige Größenordnung von mindestens 99% Umsatz nicht erreicht wird.

### Ausführungsbeispiele

### Beispiel 1

255,7 g (1,00 mol) 5-Chlorcarbonyl-5H-dibenz/b,f/azepin (II) werden in 1400-1500 ml Toluen suspendiert . Unter Rühren heizt man die Suspension auf 80-90°C an. In die heiße Lösung leitet man NH₃-Gas ein. Das Reaktionsgefäß bleibt dabei geschlossen.

Das eingeleitete NH₃-Gas reagiert sofort weg unter Bildung von Carbamazepin und NH₄Cl. Die Einleitung wird so einreguliert, daß im Labormaßstab kein wesentlicher Druckanstieg über 1,0 kPa erfolgt. Nach ca. 30 Minuten beginnt die Ausscheidung des Carbamazepins. Nach ca. 1 Stunde ist die Mischung stark kristallin, aber noch gut rührfähig. Im Verlauf der nächsten 30-60 Minuten wird die Suspension zunehmend dicker und schlechter rührfähig. Die Masse wird quarkartig dick. Im Labor rührt ein Säbelrührer oder ein Turbinenrührer die Masse nur noch in der Mitte auf. Nach Einleitung von ca. 100 % der theoretischen NH₃-Menge wird eine Stunde bei 80-95°C nachgerührt. Anschließend werden bei ca. 90°C 100 ml Wasser zugesetzt. Die Einleitung von NH₃ wird fortgesetzt, wobei die Temperatur der Reaktionsmischung bei 80-85°C gehalten wird. Nach ca. 0,5 - 1,0 Stunden wird die quarkartige Suspension immer besser rührfähig . Es entsteht eine sandartig-kristalline , schwere Kristallmodifikation, die beim Abstellen des Rührers absinkt. Unter diesen Bedingungen reagiert das in Lösung befindliche 5-Chlorcarbonyl-5H-dibenz/b,f/azepin innerhalb von 3- 5 Stunden zu mindestens 99 % zum Carbamazepin durch.

Zur Endpunktbestimmung (DC) wird nach Abstellen des Rührers die sich oben abscheidende klare Toluenlösung verwendet. Nach Beendigung der Amidierung wird die Reaktionsmischung auf 15-20°C abgekühlt. Das gebildetet Carbamazepin bleibt sandartig kristallin. Das Produkt wird abgesaugt. Das feuchte Kristallgemisch aus Carbamazepin und Ammoniumchlorid wird anschließend bei 50-60°C in ca. 1000ml Wasser verrührt . Nach dem Abkühlen wird das Carbamazepin abgesaugt. Der kristallkuchen wird ein-bis zweimal mit Wasser gewaschen . Das Produkt wird getrocknet.

Ausbeute: 228 -235 g rohes , beige- bis cremefarbenes 5-Carbamoyl-5H-dibenz/b,f/azepin (III), 96,5- 99,5 % der Theorie F.190 -192°C

Das rohe Carbamazepin kann bis zu 1% Ammoniumchlorid enthalten. Das Salz ist durch Umkristallisation aus Methanol-Wasser-Gemisch sehr gut entfernbar.

Aus der Toluen -Mutterlauge [die kein (!) Wasser enthält ] erhält man nach dem Einengen durch Destillation weitere 4,4 - 4,5 g 5-Carbamoyl-5H-dibenz/b,f/azepin ( ca. 1,8 - 1,9 % der Theorie), das durch 5-Chlorcarbonyl-5H-dibenz/b,f/azepin (II), 9-Methylacridin und weitere unbekannte Nebenprodukte verunreinigt ist. Die Rückstände aus der Toluen-Mutterlauge werden gesammelt. Eine größere Menge dieser Rückstände kann entweder durch Nachamidierung zu 5-Carbamoyl-5H-dibenz/b,f/azepin verarbeitet werden oder durch hydrolytische Spaltung mit Ätzkali zu Iminostilben umgesetzt werden.

Nach entsprechender Reinigung kann das auf diese Weise zurückgewonnene Iminostilben wieder in den Phosgenierungsprozeß eingesetzt werden.

### Beispiel 2

In einer 2000 l Rührmaschine mit Ankerrührer ( 63 U/min) werden 200 kg (0,78 kmol) 5-Chlorcarbonyl-5H-dibenz/b,f/azepin in 1200 l Toluen suspendiert. Die Suspension wird unter Rühren auf ca. 80°C angeheizt. Sobald eine klare Lösung vorliegt, beginnt man mit der Zudosierung von NH₃. Die Temperatur der Reaktionsmischung steigt in Abhängigkeit von der Geschwindigkeit der NH₃-Zugabe auf 95-105°C. Der Druck im Reaktionsapparat liegt in Abhängigkeit von der Temperatur und der Geschwindigkeit der NH₃-Dosierung bei 0,01 - 0,15 MPa (Überdruck).Nach der Einleitung von 30 kg NH₃ verrührt man 1-2 Stunden bei 80-100°C. Anschließend gibt man bei ca 80-85°C 50 l Wasser hinzu und drückt 2-5 kg NH₃ nach. Man verrührt eine weitere Stunde bei 80-85 °C. Die Reaktionsmischung wird zusehends besser rührfähig . Zur Vervollständigung der Amidierung hält man die Reaktionsmischung noch 2-5 Stunden unter Rückfluß.

Nach der positiven Endpunktbestimmung wird die Suspension abgekühlt und zentrifugiert. Das Produkt wird auf der Zentrifuge einmal mit Toluen gewaschen.

Ausbeute : 331 kg Rohprodukt ( Carbamazepin, Ammoniumchlorid, Wasser, zentrifugenfeucht)

Das feuchte Rohprodukt wird in 1000 l Wasser bei 50-60°C verrührt. Nach dem Abkühlen wird das Carbamazepin über eine Zentrifuge abgetrennt. Das Produkt wird auf der Zentrifuge mit Wasser gewaschen.
Ausbeute: 240 - 255 kg zentrifugenfeuchtes Rohcarbamazepin, 180 kg trocken, 97,4 % der Theorie
F. 189-192°C
Aus der Toluen-Mutterlauge erhält man weitere 3- 4 kg Rohcarbamazepin (1,6-2,2% d.Th.)

### Beispiel 3

In einer 1600 l Rührmaschine mit Impellerrührer ( 125 U/min) werden 230 kg ( 0,9 kmol) 5-Chlor-carbonyl-5H-dibenz/b,f/azepin in 1200 l Toluen suspendiert.

In die heiße Lösung ( ca. 85°C) wird NH₃ dosiert. Nach der Einleitung von 20 kg NH₃ ist die Reaktionsmischung durch das ausgeschiedene Carbamazepin-Ammoniumchlorid-Gemisch schon sehr dick. Die Temperatur steigt ohne Heizung auf 102 - 105 °C an, und der Druck steigt bis auf eine Atmosphäre überdruck.

Insgesamt werden 37 - 38 kg NH₃ eingeleitet. Anschließend wird noch 1-3 Stunden bei ca. 85 - 95 °C gerührt.

Die Reaktionsmischung wird über eine Absorptionsanlage entspannt. Bei ca. 80-85°C werden 50 l Wasser zugesetzt. Die Suspension wird innerhalb einer Stunde immer besser rührfähig. Man hält die Mischung weitere 2-5 Stunden bei leichtem Rückfluß. Die Endpunktbestimmung erfolgt dünnschichtchromatographisch .

Die Aufarbeitung wird nach Beispiel 2 vorgenommen.
Ausbeute: 204-208 kg trockenes Rohcarbamazepin, 95,5 -98 % der Theorie
F. 189-191°C

### Beispiel 4

In einer 1600 l Rührmaschine mit Impellerrührer ( 125 U/ min) werden 230 kg ( 0,9 kmol ) 5-Chlorcarbonyl-5H-dibenz/b,f/-azepin in 1200 l Toluen bei 85-105 °C mit NH₃ unter Druck amidiert. Die Arbeitsweise entspricht Beispiel 3. Der Druck kann bis auf 0,3 MPa ansteigen.

Nach der Einleitung von 37-38 kg NH₃ wird noch 1 Stunde bei 85-105 °C gerührt. Danach wird die Reaktionsmischung auf ca. 80°C abgekühlt und über eine Absorptionsanlage entspannt.

Bei ca. 80°C werden 50 l 10-15%iges Ammoniakwasser zugesetzt. Innerhalb von 1 Stunde tritt durch das zugesetzte Wasser eine Kristallstrukturumwandlung ein. Man hält die Reaktionsmischung bis zum vollständigen Umsatz (dünnschichtchromatographische Bestimmung) weitere 2-5 Stunden bei leichtem Rückfluß. ( aceotroper Siedepunkt ist etwas erniedrigt durch NH₃, Absorptionsanlage verwenden!). Die Aufarbeitung erfolgt wie in Beispiel 2 und 3 beschrieben.
Ausbeute : 200-208 kg trockenens Rohcarbamazepin 93,5 -98% der Theorie,
F. 189-192°C

Aus der Toluen-Mutterlauge kann das restliche Produkt isoliert werden.(Siehe Beispiel 1)

### Beispiel 5

In einer 3200 l Rührmaschine mit Impellerrührer ( 100-125 U/min) werden 300 kg (1,56 kmol) Iminostilben (I) in 1500 l Toluen suspendiert und nach dem vorgeschlagenen Verfahren WP C07D 320 612 phosgeniert. Nach der Abtrennung der wässrigen Phase kann gegebenenfalls auf pH 5-6 abgepuffert werden. Durch aceotrope Destillation wird das Wasser entfernt.

Die heiße Lösung des 5-Chlorcarbonyl-5H-dibenz-/b,f/ azepins (II) wird anschließend mit Toluen verdünnt ( so daß die gebildeten ca. 400 kg 5-Chlorcarbonyl-5H-dibenz/b,f/azepin in ca. 2300 bis 2500 l Toluen vorliegen) und mit 5 kg Aktivkohle versetzt. Die kohlefrei filtrierte Lösung wird in einer Rührmaschine mit Impellerrührer vom gleichen Typ bei 80-105°C und unter Druck, vorzugsweise bei 0,01 -0,25 MPa, nach Beispiel 2 bis 4 amidiert und aufgearbeitet.

Die Isolierung des trockenen, salzfrei gewaschenen Rohcarbamazepins kann über eine Rührwerkdrucknutsche erfolgen.
Ausbeute : 337-350 kg trockenes Rohcarbamazepin 94,4-98 % der Theorie
F. 189-190°C

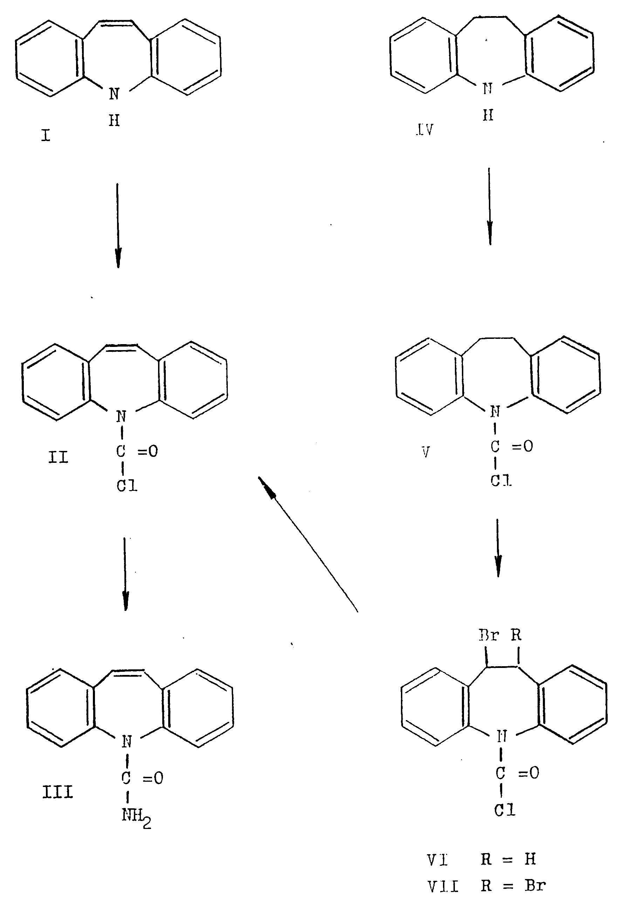

## Patentansprüche

1. Verfahren zur Herstellung von 5-Carbamoyl-5H-dibenz/b,f/azepin, indem man Iminostilben in einem inerten aromatischen Lösungsmittel wie Toluen oder Chlorbenzen bei 20 bis 60°C mit Phosgen umsetzt, aus dem neben 5-Chlorcarbonyl-5H-dibenz/b,f/azepin sich bildenden Iminostilben-Hydrochlorid durch Zugabe geeigneter wässriger Basen Iminostilben freisetzt, dieses unter Beibehaltung saurer Reaktionsbedingungen vollständig phosgeniert , anschließend bei 80 bis 90°C in Gegenwart der stark salzsauren wässrigen Phase das überschüssige Phosgen durch Hydrolyse entgiftet, die heiße wässrige salzsaure Phase abtrennt , den pH-Wert auf 5 bis 6 abpuffert, Aktivkohle zusetzt und danach die kohlefrei filtrierte Lösung des 5-Chlorcarbonyl-5H-dibenz/b,f/azepins durch aceotrope Destillation entwässert und die organische Phase mit Ammoniak amidiert, dadurch gekennzeichnet, daß man die entwässerte organische Phase mit dem gleichen Lösungsmittel verdünnt , bei 70 bis 105 °C und unter erhöhtem Druck Ammoniak bis zur Aufnahme von ungefähr 100-110 Mol% der theoretisch erfoderlichen Menge einleitet, dann nach einer Nachreaktionszeit von ca. 1 Stunde 10 bis 40 Gewichtsprozent Wasser - bezogen auf die in der Lösung enthaltene Menge von 5-Chlorcarbonyl-5H-dibenz/b,f/azepin- zusetzt, 15 bis 25 Mol% Ammoniak nachdrückt und zum Schluß weitere 3-5 Stunden, gegen Ende unter leichtem Rückfluß, bei 80 bis 95°C bis zur Vervollständigung der Reaktion rührt.

2. Verfahren nach Anspruch 1 dadurch gekennzeichnet, daß nach der Einleitung von 100 bis 110 Mol-% der theoretisch erforderlichen Menge Ammoniak vorzugsweise 25 Gewichtsprozent Wasser bezogen auf die eingesetzte Menge 5-Chlorcarbonyl-5H-dibenz/b,f/azepin entsprechend 3,5 mol Wasser auf 1,0 mol 5-Chlorcarbonyl-5H-dibenz/b,f/azepin zugesetzt werden.

3. Verfahren nach Anspruch 1 und 2, dadurch gekennzeichnet, daß an Stelle der begrenzten Wassermenge auch die gleiche begrenzte Volumenmenge verdünntes Ammoniakwasser zugesetzt wird.

4. Verfahren nach Anspruch 1 bis 3, dadurch gekennzeichnet, daß die Reaktionsmischung nach dem Zusatz der begrenzten Menge von Wasser bzw. verdünntem Ammoniakwasser und dem Nachdrücken von Ammoniak weitere 3-5 Stunden bei 80-95°C verrührt und gegen Ende unter leichtem Rückfluß über eine Absorptionsanlage entgast wird.

5. Verfahren nach Anspruch 1 bis 4, dadurch gekennzeichnet, daß über die theoretische Ammoniakmenge hinaus zusätzlich noch 15-30 Mol-% Ammoniak eingeleitet werden.

## Claims

1. Process for the preparation of 5-carbamoyl-5H-dibenz-[B,F]-azepine, wherein iminostilbene is reacted in an inert aromatic solvent such as toluene or chlorobenzene at 20°C to 60°C with phosgene, by addition of suitable aqueous bases iminostilbene is released from the iminostilbene hydrochloride produced in addition to 5-chlorocarbonyl-5H-dibenz-[B,F]-azepine, this iminostilbene is completely phosgenated under acidic reaction conditions, subsequently the excess phosgene is detoxified by hydrolysis at 80°C to 90°C in the presence of the strongly acidic aqueous phase containing hydrochloric acid, the hot aqueous phase containing hydrochloric acid is separated off, the pH value is buffered at 5 to 6, activated carbon is added and then the carbon-free filtered solution of 5-chlorocarbonyl-5H-dibenz-[B,F]-azepine is dehydrated by azeotropic distillation and the organic phase is amidated using ammonia, characterised in that the dehydrated organic phase is diluted with the same solvent, ammonia is passed in at 70°C to 105°C and under increased pressure until about 100 to 110 mol-% of the theoretically required quantity has been taken up, then after a period of approximately 1 hour after the reaction 10 to 40 per cent by weight of water - based on the quantity of 5-chlorocarbonyl-5H-dibenz-[B,F]-azepine contained in the solution - is added, a further 15 to 25 mol-% ammonia is added under pressure and finally the reaction mixture is stirred for a further 3 to 5 hours, towards the end under gentle reflux, at 80°C to 95°C, until the reaction is complete.

2. Process according to claim 1, characterised in that following the introduction of 100 to 110 mol-% of the theoretically required quantity of ammonia, preferably 25 per cent by weight of water, based on the quantity of 5-chlorocarbonyl-5H-dibenz-[B,F]-azepine used, corresponding to 3.5 mol water to 1.0 mol 5-chlorocarbonyl-5H-dibenz-[B,F]-azepine, is added.

3. Process according to claims 1 and 2, characterised in that instead of the limited quantity of water the same limited volumetric quantity of dilute ammonia water is added.

4. Process according to claims 1 to 3, characterised in that after the addition of the limited quantity of water or of dilute ammonia water and after the further addition of ammonia under pressure, the reaction mixture is stirred for a further 3 to 5 hours at 80°C to 95°C and towards the end is degassed under gentle reflux over an absorption unit.

5. Process according to claims 1 to 4, characterised in that in addition 15 to 30 mol-% ammonia, over and above the theoretical quantity of ammonia, is passed in.

## Revendications

1. Procédé pour la préparation de 5-carbamoyl-5H-dibenz/b,f/azépine, dans lequel on met en réaction de l'iminostilbène dans un solvant aromatique inerte tel que le toluène ou le chlorobenzène à 20 jusqu'à 60°C avec du phosgène, à partir de ceci en plus de la 5-chlorocarbonyl-5H-dibenz/b,f/azépine, le chlorhydrate d'iminostilbène se formant par addition de bases aqueuses appropriées libère l'iminostilbène, celui-ci dans des conditions de maintien de réaction acide phosgénise complètement, puis à 80 jusqu'à 90°C en présence de la phase aqueuse à forte teneur en acide chlorhydrique décontamine par hydrolyse le phosgène excédentaire, sépare la phase chlorhydrique aqueuse chaude, ajuste la valeur du pH à 5 jusqu'à 6, on ajoute du carbone actif et ensuite on déshydrate la 5-chlorocarbonyl-5H-dibenz/b,f/azépine par distillation azéotrope et on amide la phase organique avec de l'ammoniac, procédé caractérisé en ce que l'on dilue la phase organique déshydratée avec le même solvant, à 70 jusqu'à 105°C et sous pression accrue, on introduit de l'ammoniac jusqu'à absorption d'environ 100 - 110 % en mole de la quantité théoriquement nécessaire, ensuite après un temps de post-réaction d'environ 1 heure, on ajoute 10 à 40 % en poids d'eau rapportés à la quantité de 5-chlorocarbonyl-5H-dibenz/b,f/azépine contenue dans la solution, on maintient en pression 15 à 25 % en mole d'ammoniac et enfin pendant 3 à 5 heures supplémentaires, on agite à la fin sous léger reflux, à 80 jusqu'à 95°C jusqu'à l'achèvement complet de la réaction.

2. Procédé selon la revendication 1, caractérisé en ce qu'après l'introduction de 100 à 100 % en mole de la quantité théoriquement nécessaire d'ammoniac, de préférence 25 % en poids d'eau, rapportés à la quantité mise en oeuvre de 5-chlorocarbonyl-5H-dibenz/b,f/azépine correspondant à 3,5 moles d'eau pour 1,0 mole de 5-chlorocarbonyl-5H-dibenz/b,f/azépine.

3. Procédé selon la revendication 1 ou 2, caractérisé en ce que qu'à la place de la quantité d'eau limitée, on ajoute également la même quantité volumique limitée d'eau ammoniacale diluée.

4. Procédé selon la revendication 1 à 3, caractérisé en ce que le mélange réactionnel après addition de la quantité limitée d'eau ou d'eau ammoniacale diluée et le maintien en pression de l'ammoniac, on agite pendant 3 - 5 heures supplémentaires à 80-85°C et vers la fin sous léger reflux et l'on procède au dégazage par l'intermédiaire d'une installation d'absorption.

5. Procédé selon la revendication 1 à 4, caractérisé en ce qu'en plus de la quantité d'ammoniac théorique, on ajoute 15-30 % en mole d'ammoniac.
